# EUROPEAN PATENT SPECIFICATION

(11) **EP 1 435 901 B1**
(45) Date of publication and mention of the grant of the patent: **26.09.2007**
(21) Application number: 02729206.9
(22) Date of filing: 15.05.2002
(51) Int. Cl.: A61K 8/60, A61Q 5/08

(54) **HAIR TREATMENT COMPOSITIONS AND METHODS**
HAARBEHANDLUNGSZUSAMMENSETZUNGEN UND -VERFAHREN
COMPOSITIONS DE TRAITEMENT DU CHEVEU ET PROCEDE ET APPAREIL ASSOCIES

(30) Priority: 15.05.2001 US 290886 P
(43) Date of publication of application: 14.07.2004
(73) Proprietor: Evans, Roy M., Jr., Memphis, TN 38119 (US); Moore, Edward R., Panama City Beach, FL 32413 (US)
(72) Inventor: Evans, Roy M., Jr., Memphis, TN 38119 (US); Moore, Edward R., Panama City Beach, FL 32413 (US)
(74) Representative: Sanderson, James L.C.
(86) International application number: PCT/US2002/015288
(87) International publication number: WO 2002/092036

(56) References cited:
- US-A- 5 639 451
- US-A- 5 885 561
- US-A- 6 103 223
- US-A- 6 116 250
- US-B1- 6 171 347
- DATABASE CHEMABS CHEMICAL ABSTRACTS SERVICE, COLUMBUS, OHIO, US; XP002293221 retrieved from STN Database accession no. 25: 32 977 & W.J. WILSON ET AL.: "Further experience of the bismuth sulfite media in the isolation of E. typhi ans S. schottmulleri from feces, sewage and water" JOURNAL OF HYGIENE, vol. 31, 1931, pages 138-161,
- M.L. SCHLOSSMANN ED.: "The chemistry and manufacture of cosmetics: formulating. Vol.II, 3rd edition." 2000, ALLURED PUB. , USA 277870 , XP002293220 * page 568 *

## Description

The present invention relates to methods and compositions for the treatment of hair, and more particularly to methods and compositions for altering the shape and/or color of hair.

### Hair Coloring Art

Hair treatment compositions are frequently used to alter the color of hair. The capability of precisely producing the desired degree of color change is of primary importance for all hair coloring techniques. However, the ability to fully achieve this objective has heretofore been limited by countervailing requirements. For example, hair coloring treatments must be effectively completed in the shortest possible period of time in order to be acceptable to the person undergoing the treatment; yet, longer treatment times have heretofore typically been required to achieve the frequently desired deep tones of color. Moreover, many of the components of the compositions used to alter hair color are known to produce offensive skin and/or scalp irritation, especially when used in concentrations adapted to effect the maximum change in color. From the viewpoint of the hair care professional, it is also highly desirable that the treatment techniques be as simple as possible; yet, one-step methods for dyeing hair have heretofore been rare and/or relatively ineffective.

In general, the color of human hair has heretofore been altered either by bleaching, dyeing or a combination thereof. Mammalian fibers, including human hair, are composed of three major components: a cuticle, a cortex and a medulla. The medulla is central to the hair shaft and is wrapped by strands of keratin, which forms the cortex. The cuticle consists of overlapping flat scales covering the cortex. Melanin is the principal pigment responsible for the color of human hair. Chemical bleaching alters hair coloration by the removal and/or alteration of the melanin. This is typically accomplished by applying oxygen releasing compounds to the hair. Perhaps the most widely used of such compounds is hydrogen peroxide, which is commonly applied in the form of an aqueous solution. Such hydrogen peroxide solutions operate by opening the imbrications of the cuticle, penetrating and attacking the keratin structure and gradually lightening the shade of the hair by oxidizing the melanin. The "lightening" of the hair increases as contact times and hydrogen peroxide concentrations increase. According to prior bleaching methods, however, these conditions also tend to produce scalp irritation and undesirable weakening of the hair shaft. Furthermore, modifying the color of human hair with bleaching agents alone does not necessarily impart the desired color or shade of hair. In particular, bleaching of hair with oxidizing agents alone often results in hair having a "washed out" appearance.

Another method for altering the color of hair involves dyeing the hair. In most hair dyeing techniques, synthetic organic agents are applied to the hair to either temporarily or permanently add color thereto. Examples of temporary or semi-permanent dyes include, for example, azo and nitro compounds, and derivatives of naphthalene and anthraquinone. Semi-permanent dyes are direct dyes and generally do not require any bleaching action to color the hair. However, semi-permanent dyes generally only remain on the hair temporarily and are gradually washed out by successive ordinary shampoos.

Permanent dyes, on the other hand, comprise oxidation dyes, also known in the art as peroxide dyes. Most of these dyes comprise synthetic organic compounds which generally require an amount of hydrogen peroxide or some other non-contaminating compound that readily liberates oxygen for the development of the color on the hair. The compounds frequently referred to as oxidation dyes are more properly referred to as dye intermediates, because their actual dyeing properties are developed only upon oxidation. While a large number of compounds posses the potential for being used as dye intermediates for in vivo hair coloring, only a few such compounds have been used according to prior techniques. For example, many nitro and alkyl compounds posses desirable dyeing properties but have been unavailable for use because they are known to irritate the skin. See Wall, F. E., "Bleaches, Hair Colorings, and Dye Removers," Cosmetics: Science and Technology, Vol. 2, 2nd ed., John Wiley & Sons, p. 307 (1972).

Modification of hair color using a dyeing agent alone also frequently produces less than the desired outcome. For example, the prior hair dyeing techniques are known to frequently result in hair having an unnatural "painted" or "brassy" appearance. This undesirable result has previously been overcome by lightening the hair prior to dyeing by exposing the hair to a bleaching operation. However, this complicates and lengthens the coloring procedure,

To avoid this two-step procedure, compositions containing both a bleaching agent and a dyeing agent have been proposed. These compositions generally contain hydrogen peroxide as both the bleach and the developing agent. These bleach-dye combinations suffer from serious drawbacks. In particular, exact proportions of ingredients are usually required such that a precise amount of oxygen is released to ensure that the hair is bleached while the dye is entering it, Furthermore, excessive release of oxygen results in bleaching of the dye itself See Wall, F. E., "Bleaches, Hair Colorings, and Dye Removers," Cosmetics: Science and Technology, Vol. 2, 2nd ed., John Wiley & Sons, pp. 279-343 (1972).

U.S. Patent No. 5,560,750 discloses hair coloring compositions which incorporate non-reducing disaccharides such as sucrose to improve the hair coloring properties of the dye component and to protect keratin fibers in the hair from damage.

### SUMMARY OF THE INVENTION

Accordingly, it is an object of the present invention to provide hair treating compositions and hair treatment processes which produce desirable color alteration, preferably in relatively short periods of time.

It is a further object of the invention to provide hair treatment compositions which are relatively non-irritating to the skin.

It is a still further object of the invention to provide hair treatment compositions which do not cause unwanted damage to the hair and are readily adaptable for in vivo use.

It is another object of certain embodiments of the invention to provide effective hair coloring processes that provide bleaching and dyeing in a single step.

Applicants have discovered that these and other objects of the present invention are achieved by treating compositions, and especially hair coloring compositions, comprising a treating agent, especially a coloring agent, and a reducing saccharide. According to preferred embodiments, the reducing saccharide comprises glucose and the coloring agent comprises at least one oxidizing agent for bleaching the hair and/or developing the dyeing agent, and a dyeing agent for adding color to the hair. Such compositions have been found capable of producing exceptionally deep and desirable color, even without pre-bleaching. Furthermore, such compositions have surprisingly been found to be effective after only relatively short periods of application while being exceptionally non-irritating to the skin and scalp, and non-damaging to the hair.

The present invention permits a process for altering the color of hair to a predetermined degree comprising (a) applying to the hair a hair coloring composition, said composition comprising a coloring agent and a reducing saccharide, preferably glucose, (b) allowing said composition to remain in contact with the hair for a time sufficient to achieve the predetermined degree of color alteration; and (c) substantially removing said composition from the hair.

Furthermore, in accordance with certain aspects of the present invention, it has now been found that the properties ofthe hair treatment compositions disclosed U.S. Patents Nos. 4,947,878, 5,101,84Z, 5,415,856, 5,560,750 and 5,639,451 can be improved by the use of a reducing saccharide in place of the non-reducing disaccharides disclosed therein when the compositions comprise highly caustic components. The reducing saccharides protect the hair and scalp from the caustic component, improve performance (especially in applications relating to changing the color of hair), and control the unacceptable odor heretofore associated with such hair care compositions.

Preferred reducing saccharides for use in the present invention include disaccharides such as maltose or lactose, and monosaccharides such as glucose, with glucose being generally preferred.

### DETAILED DESCRIPTION OF THE INVENTION

### I. CHEMISTRY OF HAIR

In order to more fully understand the compositions and methods of the present invention, it is helpful to understand the basic structure of hair. Hair is a complex organic substance consisting largely of the protein keratin. More specifically, hair is a proteinaceous fiber comprising a bundle of long individual protein molecules which are intertwined with one another and cross linked at various intervals. Each individual protein molecule comprises condensed amino acids in which the acid end of one molecule is condensed with the amino end of the next. The amino acids are alike in that they all contain an acid group and an amino group, but they may not be alike in certain other details of the arrangement of their atoms. Hair protein generally contains from about 5% to about 15% by weight of the amino acid cystine, which has the empirical chemical formula C₆H₁₂N₂O₄S₂ and generally conforms to the molecular chemical formula given below: 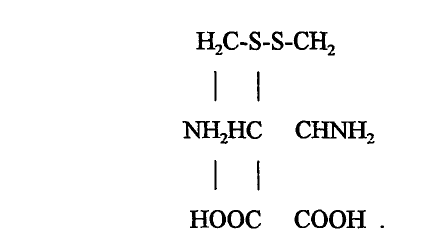 It has been postulated that cystine frequently appears in the fibrous bundle of keratin protein molecules as a bridge between adjacent peptide chains and that it may also frequently appear as a loop or bridge between two segments of the same peptide chain. It is believed that these cystine bridges affect, and in large part determine, the physical shape and conformation of the hair. It has also been postulated that links between adjacent peptide chains may also occur by the ionization of the carboxyl and amino groups to form a salt bridge. Hydrogen bonding is believed to provide a third means by which linkage between adjacent peptide chains may be achieved. It is believed that these additional linkage mechanisms also affect the physical shape and conformation of the hair. See chapter 26 of the book "Chemical and Manufacture of Cosmetics" by Mason G. Denavarre and the first chapter of the book entitled "The Proteins Volume 4", Third Edition, edited by Hans Nuroff and Robert L. Hill, 1979.

### II. The Coloring Compositions

The present coloring compositions comprise two important ingredients: coloring agent and reducing saccharide. Applicants have found that the inclusion of reducing saccharide, and preferably monosacharides such as glucose, in compositions according to the teachings of the present invention provides the compositions with highly desirable and unexpected properties. In particular, the present compositions have been found to be non-damaging to the hair as compared to prior products. Furthermore, the compositions when used in hair color lightening applications have been found to produce exceptional "lift" to the hair. More particularly, the present composition have been unexpectedly found to produce a full 35% percent improvement in "lift." As is known to those skilled in the art, "lift" refers to the degree of lightening that is imparted to hair color as a result of application of the composition.

Furthermore, the present compositions have unexpectedly been found to produce exceptionally deep color to the hair. Although applicants do not wish to be bound by or to any particular theory of operation, it is believed that the synergistic combination of ingredients used in the present invention prevents or at least inhibits autooxidation of the dye molecules contained in the composition, thus tending to color the hair deeply, as opposed to only on the surface of that hair as is apparent with certain prior art products. According to certain embodiments, this desirable color alteration is achieved without the benefit of a pre-bleaching step.

### A. The Coloring Agents

The present compositions comprise coloring agent, preferably a major proportion of coloring agent, and more preferably from about 85 to about 99 percent by weight of coloring agent and more preferably from about 95 to about 99 percent by weight of coloring agent. The term coloring agent is used herein in a non-limiting sense to refer to any agent, compound or composition adapted to alter the color of hair. Thus, the coloring agents of the present invention include those compositions which tend to remove color from the hair as well as those which add color.

It is contemplated that the coloring agents of the present invention will generally include one or more active color compounds and carrier for the active compounds. It will be appreciated that the terms active color compound and carrier are used herein for the purpose of convenience and illustration but not necessarily by way of limitation. In particular, the term active color compound refers to those components of the coloring agent which interact chemically or physically with the hair or with other components of the coloring agent to add or remove color from the hair. In contrast, the carrier serves to provide the proper environment for the active compounds and to facilitate, enhance and/or modify delivery and application of the active compounds to the hair. The preferred coloring agents of the present invention comprise from about 3 to about 20 percent by weight of active coloring compounds and from about 80 to about 95 percent by weight of carrier, and even more preferably from about 5 to about 15 percent by weight of active coloring compound and from about 80 to about 90 percent by weight of carrier.

An important aspect of the present invention resides in the weight ratio of reducing saccharide to active coloring compounds. Applicants believe that such ratio may vary widely, depending upon numerous factors, such as the type of hair and the degree of color alteration desired. It is preferred, however, that the reducing saccharide: active coloring compound weight ratio be from about 0.01:1 to about 1:1, and even more preferably from about 0.02:1 to about 0.05:1. Applicants have found that, according to certain embodiments, ratios of less than about 0.01:1 produce compositions which exhibit a decreased ability to protect the hair and skin of the user, while compositions having ratios greater than about 1:1 may exhibit a decreased ability to produce the desired color alteration.

### 1. Active Compounds

According to certain embodiments of the present invention, the coloring agent comprises an oxidizing agent. As will be appreciated by those skilled in the art, oxidizing agents are commonly used as active components for both removing color from and adding color to hair. As the term is used herein, oxidizing agent refers to those compounds which are oxidizing agents with respect to melanin in the hair or with respect to the dye intermediate used in the oxidative dyeing agents of the present invention. For example, in bleaching operations the oxidizing agent attacks the melanin of the hair and removes color, thereby lightening the hair color. In dyeing operations, an oxidizing agent is typically included to aid in the conversion of the dye intermediates used in permanent dyeing operations. It is seen, therefore, that the amount of oxidizing agent contained in the present compositions will vary widely, depending upon the particular circumstances of each embodiment. It is generally preferred, however, that the present compositions comprise from about 3 to about 20 percent by weight of oxidizing agent. For embodiments in which bleaching and dyeing steps are carried out separately, the compositions preferably comprise from about 10 to about 20 percent by weight of oxidizing agent for bleaching compositions and from about 1.5 to about 15 percent by weight, and even more preferably from about 1.5 to about 10 percent by weight, of oxidizing agent for dyeing compositions. For certain preferred embodiments in which bleaching and dyeing are carried out using a single composition, the compositions preferably comprise from about 4 to about 10 percent by weight of oxidizing agent and even more preferably from about 5 to about 9 percent by weight of oxidizing agent.

The oxidizing agent to be used in accordance with the present invention may comprise any of a number of conventional or unconventional oxidizing agents. Generally, it is only required that the oxidizing agent be nontoxic, mild in action and free of harmful residue. For the removal of color from the hair, such as in bleaching operations, it is preferred that the oxidizing agent comprise a salt of persulfuric acid (H₂S₂ O₈), and preferably alkali metal and ammonium salts of persulfuric acid. It is especially preferred for bleaching operations that the oxidizing agent be selected from the group consisting of sodium persulfate (Na₂S₂O₈), potassium persulfate (K₂S₂O₈), ammonium persulfate ((NH₄)S₂O₈), and mixtures of two or more of the these. Other suitable oxidizing agents, such as peroxides, for use in bleaching operations, in addition to those exemplified above, will be apparent in view of the present disclosure.

According to other embodiments of the present invention, the hair coloring composition comprises a dyeing agent for adding color to the hair. It is contemplated that the types of dyeing agent suitable for use in accordance with the present invention are numerous and varied, including temporary, semi-permanent and permanent dyes generally known to those skilled in the art. Examples of semi-permanent dyes include, for example, azo and nitro compounds and derivatives of naphthalene and anthraquinone. Such dyeing agents are generally referred to as "non-oxidative" dyeing agents, since these dyeing agents do not require oxidation to color the hair.

It is generally preferred, however, that the dyeing agent of the present invention comprise an oxidative dyeing agent. As the term is used herein, oxidative dyeing agent refers to those dye intermediates or precursors which produce color upon oxidation. The oxidative dyeing agents of the present invention preferably comprise monomeric aromatic compounds which, on oxidation, form oligomers or polymers having extended conjugated systems of electrons in their molecular structure. This oxidative reaction produces oligomers and polymers with electronic structures in the visible spectrum. As a result, oxidation of the dye intermediates or precursors results in the development of color. Especially preferred oxidative dying agents comprise substituted phenols, amino phenols, diamines, including the o-and p-diamines, aminohydroxy compounds of benzene, and derivatives of these which pass through a quinoid stage during oxidation. According to certain embodiments, aromatic amines having two functional groups, such as p-phenylenediamine, are preferred for their ability to yield higher molecular weight colored materials upon oxidative polymerization. Mono functionalized aromatic amines capable of yielding colored conjugated imines, and quinoid dimers, trimers, etc. are preferred according to other embodiments. It is also contemplated that combinations of these various dye precursors may be used.

Suitable oxidative dyeing agents to be used in accordance with the present invention are disclosed, for example, in U.S. Pat. Nos. 4,473,375 and 4,840,639 and F. E. Wall, "Bleaches, Hair Colorings, and Dye Removers," Cosmetics: Science and Technology, Vol. 2, pp. 300-320 (1972). Other dyeing agents to be used in the present invention would be readily apparent to one skilled in the art.

For embodiments comprising an oxidative dyeing agent, its highly preferred that the compositions include an oxidizing agent comprising hydrogen peroxide. It will be appreciated by those skilled in the art that such oxidizing agents operate to develop the color of the dye intermediate and that other developing agents, either alone or in combination with the present oxidizing agents, may be used within the scope ofthe present invention. What is required in such embodiments is that the developing agent, which is preferably an oxidizing agent, converts the synthetic organic hair coloring intermediates to the desired color.

Applicants have found that the present invention provides a surprising and beneficial result with respect to the amount of oxidizing agent required to develop the color of commonly used oxidative dyeing agents. It has heretofore been common practice to use a 6 wt. % aqueous solution of hydrogen peroxide for developing dye intermediates. Furthermore, it has also been common practice to employ about 2 parts by volume of such hydrogen peroxide solution for every one part by volume of dye base (i.e., dyeing agent plus carrier). In contrast, certain embodiments of the present invention utilize as little as 0.5 parts by volume of hydrogen peroxide solution (6 wt, %) for each part by volume of dye base without any noticeable decrease in hair color alteration, In fact, applicants have discovered that even with this reduced ratio, hair color alteration is effected in substantially shorter time periods than those required by the prior art.

### 2. The Coloring Ageats-Carrier

It will be appreciated by those skilled in the art that many of the active compounds described herein are,most readily available in the form of dispersions or solutions of one or more liquids, typically aqueous solutions. It is contemplated the active ingredients of the present invention will frequently be utilized in this from, and accordingly the present compositions preferably include a carrier for the coloring agent. In general it is contemplated that a wide variety of materials will be suitable for use as a carrier, and all such materials are within the scope of the present invention. It is generally preferred that the carrier comprise a liquid, preferably a polar liquid, for facilitating delivery and application of the present coloring agent to the hair. As will be appreciated by those skilled in the art, the physical condition of the carrier may therefore vary widely, ranging, for example, from a thin clear liquid to a creamy paste, depending upon the needs of the particular application.

The carrier of the present invention preferably comprises a solvent for one or more of the active components of the coloring agent. Thus, for compositions containing polar oxidizing agent and/or polar dyeing agents, the carrier preferably comprises a polar liquid, such as water, alcohol and mixtures of these. The term solvent is used in this context in a broad sense to include those liquid components and mixtures of liquid components which have at least some tendency to solubilize at least one active component of the coloring agent. It is especially preferred that the carrier comprise a mixture of water and an alcohol and/or glycol, such as C₂ - C₆ alcohols, preferably isopropyl alcohol, and C₂ - C₆ glycols, preferably propylene glycol and/or hexylene glycol. The composition preferably comprises solvent in an amount from about 40 to about 85 percent by weight of the composition.

In accordance with a preferred embodiment of the present invention, the carrier comprises a thickening agent for adjusting the rheology of the composition. The type and amount of such thickening agents may vary widely within the scope of the present invention. Thus, the thickening agents suitable for use in the present compositions are those thickening agents typically used in cosmetics and generally include organic and inorganic compounds. Examples of suitable thickening agents include silica, carboxymethylcellulose, fatty alcohols and mixtures of two or more of these. It is preferred that the fatty alcohol comprise lauryl alcohol. A suitable lauryl alcohol is CO-1214, commercially available from Procter & Gamble of Cincinnati, Ohio. A suitable carboxymethylcellulose is CMC-7H3 SF, commercially available from Hercules, Inc. of Wilmington, Del. The carrier preferably comprises an amount of the thickening agent to provide the aqueous composition with the desired thickness or viscosity. It is generally preferred, however, that the present coloring composition comprises from about 5 to about 15 percent by weight of thickening agent, with about 8 to about 12 weight percent being even more preferred in certain embodiments..

For embodiments in which the composition includes a dyeing agent, and particularly an oxidative dyeing agent, the carrier also preferably comprises an alkalizer or buffer for providing the proper environment for the dye intermediate, as is well known in the art. In certain embodiments the carrier preferably comprise an aqueous solution, and preferably a 28 wt. % solution, of ammonium hydroxide as alkalizer. In other embodiments, the alkalizer comprises sodium borate and/or urea. The amount of alkalizer will of course depend upon the particular dyeing agent and other factors. It is generally preferred, however, that the compositions of the present invention include from about 5 to about 10 percent by weight of 28 wt. % aqueous ammonium hydroxide solution, or from about 0.5 to about 5 percent by weight of urea (GR prilled from EM Science)..

Embodiments including dyeing agent also preferably include spreading agent to assist in distribution of the dyeing agent evenly along the hair shaft. Suitable spreading agents include most well known surfactants, such as ethoxylated alkylphenols, and preferably octylphenoxy poly(ethyleneoxy)ethanol, which is commercially available as Igepal CA-630 from Rhone-Poulenc/GAF of Wayne, N.J. Spreading agents in form of polyethylene glycol octophenol ether (sold under the trade name TRITON X-100) are also preferred in certain embodiments.

The carrier also preferably comprises a detergent for leaving the hair feeling smooth and soft after treatment with the compositions of the present invention. Suitable detergents include those detergents readily known to those skilled in the art, including primary alkyl sulfates of the C 12 -C18 series, salts of oleic acid, ammonium hydroxide, zwitterionic compounds and mixtures of two or more of these. Preferably, the detergent comprises ammonium lauryl sulfate. A suitable ammonium lauryl sulfate is Emersal 633LL.sup.R, commercially available from Emery Chemicals Personal Care and Specialties Group of Linden, N.J. Examples of oleic acid and aqueous ammonium hydroxide which are readily available include Emersal 6333LL.sup.R, commercially available from Emery Industries, Inc., Fatty and Dibasic Acids Group, Cincinnati, Ohio. An example of a suitable zwitterionic compound to be used in accordance with the present invention is lauramido propyl betaine, commercially available from Mona Industries, Inc. of Patterson, N.J. Other detergents suitable for use in the carrier of the aqueous solution of the present invention would be readily apparent based upon the present disclosure.

In a preferred embodiment of the present invention, the carrier comprises chelating agent. The purpose of the chelating agent in the present compositions is to chelate or bind heavy metals which may be present in the water of the aqueous compositions. In the absence of such chelating agent, such metal ions may deleteriously affect the performance of the active color components. Accordingly, the amount and type of chelating agent will depend, for example, on the quality of the water used in the carrier and the sensitivity of the active color components. Thus, the chelating agent may comprise any of a number of conventional or unconventional chelating agents used in conventional amounts. It is preferred, however, that the chelating agent comprise, and preferably consist of, ethylenediaminetetraacetic acid ("EDTA"). An example of a suitable EDTA to be used in accordance with the present invention is Hamp-ene acid® commercially available from W. R. Grace and Co. of Nashua, N.H.

For embodiments in which the coloring agent comprises oxidative dyeing agent, it is generally preferred that the carrier include an anti-oxidant to assist in the prevention of premature decomposition of the dye intermediates. It is contemplated that customary types and amounts of anti-oxidants may be used within the scope of the present invention. Sodium sulfite and ascorbic acid are antioxidants which may be used in customary amounts in the compositions of the present invention.

### B. The Reducing Saccharide

An important aspect of the present invention is the provision of hair coloring compositions containing reducing saccharide. In particular, it is contemplated that the reducing saccharide of the present invention acts as a protecting agent for protecting the keratin fibers of the hair from unfavorable damage and degradation while also permitting, and preferably enhancing, the oxidation of melanin contained in the hair. Furthermore, applicants have found that the presence of reducing saccharide in hair treatment compositions tends to also protect the scalp of the person being treated and the hands of the hair care professional from irritation and burning. In embodiments in which the composition includes oxidative dyeing agent, the reducing saccharide appearers to also act as a protecting agent to the extent that it inhibits premature or excessive development of the dye intermediate.

An especially preferred embodiment of the present invention provides coloring compositions in which the coloring agent comprises oxidative dyeing agent and oxidizing agent, the amount and type of the oxidizing agent being effective to develop said oxidative dyeing agent and to remove color from the hair. Such embodiments are preferred for the advantage of providing a composition which is highly effective for substantially simultaneously removing and adding color to hair, a characteristic which has been long sought but not heretofore fully achieved. The difficulty encountered by prior art compositions stemmed from the conflicting and contradictory requirements of the components of such compositions. In particular, bleaching of hair to remove color has heretofore generally required a type and amount of oxidizing agent which has been detrimental to effective performance of the dye intermediates. Thus, it has heretofore been difficult if not impossible to effectively formulate compositions containing a type and amount of oxidizing agent effective to perform both lightening and development functions. Applicants have unexpectedly found that such a characteristic is possessed by certain preferred embodiments of the present compositions.

Without being bound by or limited to any particular theory of operation, it is believed that the ability of the present composition to both lighten hair and develop dye intermediates is due, at least in part, to the beneficial effects of reducing saccharide in the present compositions. In particular, it is believed that the reducing saccharide component, and preferably glucose, maltose and combinations of these two, favorably regulate development of the dye intermediate, enhances the oxidative reaction of the melanin in the hair, and moderates harmful oxidative attack on the keratin fibers in the hair. In connection with regulation of the reaction of the dye intermediates, it is contemplated that the reducing saccharide may interfere to a favorable extent with the chemical interaction of the oxidizing agent and the dye intermediate. Furthermore, it is believed that, in the absence of reducing saccharide, bleaching of hair by exposure to oxidizing agents causes a disadvantageous degradation of the keratin fibers, and that this degradation inhibits binding of the developed dye to the keratin fiber. According the present invention, it is expected that the reducing saccharide acts to preferentially favor reaction of the oxidizing agent with the melanin while simultaneously protecting the keratin fibers from degradation. Applicants have thus discovered that the inclusion of reducing saccharide, such as glucose, in compositions for the coloring of hair imparts several desirable characteristics to such compositions.

As is well known to those skilled in the art, reducing disaccharides are carbohydrates comprised of two monosaccharide units. As used herein, the term reducing saccharide refers to all known and available reducing saccharide compounds, including all stereoisomeric and enantiomeric forms thereof. While it is contemplated that all such reducing saccharides are adaptable for use in the compositions of the present invention, it is highly preferred that the reducing saccharide comprise, and preferably consist essentially of D-Glucose for embodiments involving the use of active dying agents. For embodiments involving the use of bleaching without necessarily dying, it is highly preferred that the reducing saccharide comprise, and preferably consist essentially of, maltose, and even more preferably maltose monohydrate. It is also preferred that the reducing saccharide of the present invention reduces Tollens' or Fehlings' reagent.

It is contemplated that the amount of reducing saccharide used in accordance with the present invention may vary widely, depending upon numerous factors, such as hair type and the desired color alteration. It is generally preferred, however, that the coloring compositions of the present invention comprise from about 0.1 to about 5 percent by weight of reducing saccharide, with from about 0.5 to about 3 percent by weight being more preferred, and 0.1 to about 2 percent being even more preferred according to certain embodiments. According to especially preferred embodiments in which the bleaching and dyeing agents are brought together at the time of use to form a composition in accordance with the present invention, the coloring compositions comprise about 1 to about 2 percent by weight of reducing saccharide.

As mentioned hereinbefore, the ratio of reducing saccharide to active color compounds is an important aspect of certain embodiments of the present invention. It is generally preferred that such ratio, on a weight basis, is from about 0.01:1 to about 0.1:10, and even more preferably from about 0.01:1 to about 0.1:1. For coloring compositions which do not contain oxidative dyeing agents, for example bleaching compositions, the reducing dissaccharide:active coloring compound weight ratio is preferably from about 0.1:1 to about 0.3:1. For coloring compositions which do contain oxidative dyeing agents, the weight ratio of reducing saccharide: active coloring compound is preferably from about 0.02:1 to about 0.04:1.

### II. The Kits

According to certain embodiments of the invention, the present compositions are provided in kit form and preferably comprise a first container containing a first composition for adding color to the hair, a second container containing a second composition comprising carrier components and a third container containing an oxidizing agent. In such embodiments, it is contemplated that reducing saccharide is preferably included in either the first composition or the second composition or both the first and second compositions. It is contemplated that the hair care professional or in-home user can combine the contents of the kit to produce numerous and varied compositions of the present invention, thereby having the ability to achieve a range of hair coloring effects.

The first container preferably contains a composition comprising dyeing agent, and even more preferably a composition comprising oxidative dyeing agent. For the purpose of convenience, first compositions which contain dyeing agent are sometimes referred to herein as dye base. It is preferred that the first composition of the kit of the present invention is itself a composition according to the present invention. That is, it is preferred that the first composition also contain reducing saccharide. In such embodiments, the composition in the first container preferably comprises from about 0.2 to about 5 percent by weight of oxidative dyeing agent, from about 1 to about 5 percent by weight of reducing saccharide and about 85 to about 95 percent by weight of carrier.

The second composition, which is also preferably a composition of the present invention comprising carrier components and reducing saccharide, is sometimes referred to herein as bleach oil. The bleach oil preferably consists essentially of carrier components and reducing saccharide wherein the amount of reducing saccharide is preferably from about 3 to 5 percent by weight of the bleach oil. Other ingredients adaptable for use in the present compositions to effect the handling, rheology, etc., as described hereinbefore, may also be included in the composition in the second container.

The third container contains a composition, and preferably a composition in solid powder form, comprising oxidizing agent. The oxidizing agent is preferably selected so as to remove color from the hair. Thus, for the purpose of convenience, the third composition is sometimes referred to herein as bleach booster powder. For example, the composition contained in the third container preferably includes oxidizing agent comprising a salt of persulfuric acid (H₂S₂O₈), and preferably alkali metal and ammonium salts of persulfuric acid, such as sodium persulfate (Na₂S₂O₈), potassium persulfate (K₂S₂O₈), ammonium persulfate ((NH₄)₂S₂O₈). Other ingredients adaptable for use in the present compositions, as described hereinbefore, may also be included in the composition of the third container.

In operation, the bleach oil is adapted to be combined with a portion of the contents of the first container, or with a portion of the contents of the third container, or with a portion of the contents of both the first and third containers to produce a composition according to the present invention. For example, the oxidizing agent in the third container is especially adapted to remove color from hair when combined with bleach oil described above. Thus, a bleaching composition according to the present invention is prepared by mixing a portion of the first and third containers hereof Furthermore, the composition in the first container is suitable for use alone or in combination with a peroxide solution to add color to hair. For embodiments in which both removal of color from and addition of color to the hair is desired, portions of the contents of all three containers may be combined.

In all cases, it is contemplated that an aqueous peroxide solution, and preferably a 20 vol solution, which is normally not part of the kit, will preferably be utilized to produce a final composition according to the present invention.

### III. The Methods

The present invention provides methods for conveniently coloring hair comprising applying a composition of the present invention to the hair, allowing said composition to remain in contact with the hair for a time sufficient to alter the color of the hair and substantially removing said composition from the hair.

According to especially preferred aspects of the present invention, the methods achieve bleaching and dyeing of hair in a one-step procedure. This method comprises applying to the hair an aqueous hair coloring composition comprising an oxidative dyeing agent, reducing saccharide, and oxidizing agent, the amount and type of the oxidizing agent being effective to develop said oxidative dyeing agent and to remove color from the hair. Such methods are highly preferred for the ability to achieve effective removal of color from hair and addition of color to hair in a single step. The composition is preferably allowed to remain in contact with the hair for a time sufficient to bleach and dye the hair to the desired degree. The composition is then removed from the hair, preferably by steps which comprise rinsing with water.

The application of the compositions of the present invention to the hair comprises application procedures generally known to those skilled in the art. For example, the hair may be dry upon application of the compositions. Conversely, the hair may be wetted with water prior to the application of the compositions. In either event, the composition is applied in an amount so that it is substantially completely and evenly distributed throughout the hair.

In accordance with the method aspects of the present invention, the composition is allowed to remain in contact with the hair for a period of time effective to color the hair by both bleaching and dyeing the hair. Preferably, the composition is allowed to remain in contact with the hair for about 5 to about 20 minutes. Applicants have found that, due in large part to the beneficial properties of the present compositions, the contact times required by the preferred methods of the present invention to achieve a desired color alteration are substantially shorter than those required by prior art procedures. Accordingly, the color altering compositions of the present invention are preferably allowed to remain in contact with the hair for no more than about 10 minutes, and even more preferably from about 5 to about 10 minutes.

After the color of the hair has been altered to the desired extent, the compositions of the present invention are preferably removed from the hair by rinsing the hair with water. Thereafter, the hair may be cut, styled and dried in any desirable manner.

In accordance with the present invention, reducing saccharides are used in combination with various caustic hair treating compositions to protect the hair and scalp from the effects of the caustic component, to improve the performance of the composition and/or to reduce the odor of the composition.

### THE REDUCING SACCHARIDE

An important aspect of the present invention is the provision of hair altering compositions containing a reducing saccharide. In particular, it is contemplated that the saccharide of the present invention acts as an anti-oxidant, and thus protect the hair and scalp from oxidation. The reducing sugar also helps promote the activity of the cysteine and cysteamine components, when present, which act by a reducing process. Furthermore, the reducing sugars help to control unpleasant smells by reducing the production of di-thiol side products which may be formed. Thus the reducing sugars accelerate the activity of the reducing agents in the hair shaping compositions, reduce oxidation of these agents both in the bottle and in use, and prevent surface oxidation of hair and scalp during use.

The bleach oil compositions of the present invention include reducing agents, such as sodium sulfite and/or trisodium phosphate. In each case the reducing sugars promote the activity of these reducing agent components, while reducing undesirable oxidation in the bottle and in use, and protecting the hair and scalp from surface oxidation.

As discussed in the '451 patent, the sugars also acts as a protecting agent for protecting the keratin fibers of the hair from undesirable damage and degradation while also permitting, and preferably enhancing, the softening of the hair. Furthermore, the reducing saccharide also protects the scalp of the person being treated and the hands of the hair care professional from irritation and burning. While applicant does not intend to be bound by or limited to any particular theory, it is believed that the reducing saccharide provides abundant sites for hydrogen bonding with the molecules which make up the hair. The availability of such sites is believed to compete for the otherwise intermolecular hydrogen bonding which is present among the protein strands. This in turn opens the tertiary or spatial structure of the protein fibers, thereby facilitating reduction of the disulfide, cystine, down the thiol, cysteine.

As is well known to those skilled in the art, saccharides are carbohydrates and as used herein the term saccharide refers to all known and available saccharide compounds, including all stereoisomeric and antiomeric forms thereof. The reducing saccharides of the present invention are characterized as "reducing" because they reduce Tollens' or Fehlings' reagents, as is well known in the art. Preferred reducing saccharides include the disaccharides maltose and lactose, and the monosaccharide glucose. Maltose has been found particularly preferable for use in connection with the compositions of the present invention. According to preferred embodiments, the reducing saccharide comprises, and preferably consists essentially of, D-(+)-glucose. Glucose products adaptable for use in accordance with the present invention are available from Corn Products International under as a family of products sold under the trade designation Cerelose® dextrose, and all such products are considered to be adaptable for use in the preferred embodiments of the present invention, with monohydrate forms being generally preferred.

It is contemplated that the amount of reducing saccharide in the compositions of the present invention, may vary widely, depending upon numerous factors, such as hair type and the desired shape or color alteration. It is generally preferred, however, as indicated above, that the composition of the present invention comprise from 0.2 to 12 percent by weight of reducing saccharide, with amounts of from 0.2 to about 5 percent by weight being more preferred, and amounts of from about 1 to about 3 percent being even more preferred.

### The Methods

The present invention also provides methods for modifying the natural conformation of existing hair. As the term is used herein, existing hair refers to fully developed excutaneous hair. As used herein, the term "natural configuration" refers to the configuration of the hair prior to being treated according to the methods of the present invention. That is, the term "natural configuration" is used for convenience only and does not limit the methods of the present invention to treatment of hair which has not been previously treated. Surprisingly and beneficially, the use of the methods and compositions of the present invention provide the capability for effective and non-damaging treatment of previously treated hair. In contrast to the methods and compositions heretofore used, the present invention achieves softening or relaxation of the hair by relatively benign repositioning of the natural constituents of the hair without causing permanent damage thereto. As a result, hair may be subjected to a plurality of treatments without being damaged to any substantial extent.

An important aspect of the methods of the present invention is application of the compositions of the present invention to the hair of the person to be treated. While many methods are known and available to those skilled in the art for the application of softening solutions and those methods may be readily adapted for use with the present compositions, it is preferred that the present compositions be directly applied to the hair. In particular, it is preferred that the application start at the scalp and move progressively outward towards the ends of the hair until the hair being treated is covered thoroughly. It will be understood by those skilled in the art that the particular application method step described above provides unique and substantial advantages over those methods generally used in the prior art. For example, application of the highly alkaline softening compositions heretofore used generally presented a serious risk of caustic burn to both the subject being treated and the person conducting the treatment (hereinafter "the operator"). Accordingly, prior methods require that precautions be taken in order to protect both the user and operator from caustic bum. For example, kits containing such solutions generally include instructions recommending or requiring that the operator wear gloves during the application step and that the skin of the treated person be protected from the solution by thick and highly viscous gels. Due to the relatively benign nature of the present compositions, such cumbersome and inconvenient precautions are not necessary. Moreover, it is imperative according to the heretofore used methods that the application period be strictly controlled and minimized so as to avoid damage to the scalp and hair of the person being treated. Overexposure of the hair to such highly caustic solutions would generally cause severe and irreversible degradation of the structure of the hair. In comparison, the benign nature constituents of the softening solutions according to the present invention eliminates the criticality of the application period and extends the maximum application period nearly indefinitely, especially for embodiments in which the active compound comprises cysteine in major proportion.

As will be appreciated by those skilled in the art, the amount of softening composition to be applied according to the present invention will vary greatly depending upon a host of individual circumstances. For example, the particular type of hair which is to be treated will have a large impact on the amount of composition to be applied. In particular, it is well known that different types of hair have varying degrees of moisture absorbency. Since softening compositions of the present invention preferably comprise an aqueous solution, the ability of the composition to effect hair softening will depend to some extent upon this property of the individual hair. Likewise, the amount of hair to be treated will also determine the application rate of the softening composition. In addition, the extent to which the natural configuration of the hair is to be modified will also impact upon the amount of the composition to be applied. Accordingly, all application rates and amounts are within the scope of the present invention. Applicant has found, however, that based primarily upon considerations of cost and convenience, that the amount of solution to be applied is preferably from about 89 ml to about 118 ml (3 ounces to about 4 ounces) and more preferably from about 104 ml to about 118 ml (3.5 ounces to about 4 ounces).

The application period for the compositions of the present invention will also vary widely depending upon a variety of individual circumstances. Importantly and surprisingly, applicant has found that exposure of the hair and scalp to certain compositions of the present invention for extended periods of time will not result in any substantial degradation of the hair or cause deleterious effects to the scalp. On the contrary, it is believed that extended exposure of the hair to the compositions ofthe present invention, especially those compositions having a pH of about 7, may tend to invigorate and revitalize the hair rather than cause the degradation thereof

While applicant does not intend to be bound by or to any particular theory, both cysteine and cystine are naturally occurring amino acids in keratin and therefore it is believed that extended exposure of the hair to compositions of the present invention comprising cysteine will tend to replenish these components of the hair, It will also be appreciated by those skilled in the art that very short application periods are also within the scope of the present invention. That is, a very short application period may be desirable when only a modest modification of the natural hair configuration is to be achieved. Accordingly, all application periods are within the scope of the present invention. Applicants have found, however, that based primarily upon convenience considerations, it is preferred to use the following application periods according to the following approximate hair types:

| Hair Type | Approximate Application Period-Minutes |
|---|---|
| Fine Hair | 10 to 12 |
| Medium Hair | 15 to 20 |
| Coarse Hair | 20 to 30 |

Another step according to the methods of the present invention comprises placing the hair in the desired configuration. Many particular techniques are well known and available in the art for placing the hair in a variety of different configurations. Although the placing step of the present invention may take place either before, during or after the application period, it is generally preferred practice when straightening kinky or curly hair to place the hair in the desired configuration only after the application period has expired. That is, the hair will generally not be manipulated during the application period. When the present invention is used in the treatment of hair having a naturally straight configuration, however, it is generally preferred that the hair be placed in the modified configuration either before or during the application of the softening composition of the present invention. It is believed that the details of the procedures used in any particular case to achieve the desired reconfiguration of the hair will be available and well known to those skilled in the art. In applications requiring curling or waving naturally straight hair, for example, it is anticipated that the methods of the present invention will include coiling or wrapping the hair around curlers or rods after the softening agent has been applied.

The methods of the present invention also include testing the effectiveness of the softening process. In certain preferred embodiments, especially those embodiments for the straightening of kinky or curly hair, the testing step of the present invention includes running a fine tooth comb through the hair and observing the resiliency of the hair. If the comb moves through the hair with the desired degree of resistance, this is an indication that the softening process has had the desired degree of effectiveness. Depending upon the particular hair type, the extent of desired straightening, and other factors, this step may last a few seconds to several minutes.

According to a preferred aspect of the present invention, the methods of the present invention further include the step of oxidizing the hair which has received the softening composition and which has been placed in the new configuration. This oxidation step can comprise exposing the hair to air or oxygen. In certain other preferred embodiments, the hair is oxidized by contacting it with a chemical oxidizing agent or neutralizer. The oxidization step of the present invention "quenches" the activity of the softening composition. That is, by exposing the softening solution to oxidation, the capacity of the composition to soften the hair is reduced or eliminated. In this way, the precise amount of softening required can be controlled. The oxidation step also aids in the replacement of the disulfide cystine bonds which help give the hair its shape. As discussed earlier, application of the softening composition causes cleavage of these disulfide bonds and, on a macroscopic scale, renders the hair relatively malleable. While not intending to be bound by or to any particular theory, applicant believes that such malleability occurs because at least a portion of the individual protein chains which make up the hair are "decoupled" and allowed to more easily move or slide relative to one another when the disulfide bonds are broken. When the hair is thus subject to the stress caused by placing the hair in the desired configuration, this stress is relieved by the movement of the individual protein chains with respect to one another. The oxidation step according to the methods of the present invention allows such disulfide bonds to be reestablished, thus effecting a permanent reconfiguration of the hair. Standard neutralizing agents are available and well known in the art and the use of all such neutralizing agents are accordingly within the scope of the present invention. Applicant has found, however, that it is preferred to select neutralizing agents from the group consisting of hydrogen peroxides and metal bromate salts, preferably potassium and sodium bromates.

Other well known hair treatment steps may be preferably used in conjunction with the method steps described above. For example, in certain preferred embodiments, the hair is shampooed prior to application of the present invention. Shampooing in this manner removes fatty acids and oils from the hair and allows enhanced penetration of the softening composition. In a like manner, it may also be preferred to condition the hair prior to the application of the softening composition. In certain embodiments, it is also preferred that the hair be rinsed with tepid water after the application period has expired. In general, the solution which is rinsed from the hair will become clear when the rinsing step is complete. More specifically, the rinsing step is expected to last approximately five minutes. In an analogous manner, it is preferred that the hair is also rinsed after the neutralization step is complete. In order to improve the longevity of the curling and straightening process, applicants have also found that it may be desirable to spray the hair after neutralization with an ammonium sulfate solution.

While many techniques are known and available for effectively producing compositions of the present invention, applicant has found that certain preparation methods are preferred for compositions in which the active shaping compound comprises cysteine in major proportion. In particular, it is preferred that cysteine be introduced into solution in the form of hydrated L-cysteine hydrochloride since such material is readily available and contains a relatively precisely known number of milliequivalents of thiol per gram. Due to the acidic nature of hydrated L-cysteine hydrochloride in solution, however, it is preferred that a metal hydroxide be added to the solution during preparation to neutralize the acid component of the L-cysteine hydrochloride. For like reason, it is preferred to add a metal hydroxide to neutralize the acid component when dissolving the cysteamine hydrochloride. Accordingly, it is preferred that potassium or sodium hydroxide be added to the solution in an amount sufficient to provide the required number of milliequivalents of hydroxide to neutralize the acid component of the L-cysteine hydrochloride. In the methods of the present invention for preparing the softening compositions thereof, it is also preferred to introduce a standard anti-oxidant into solution during the preparation process. Although such anti-oxidants are not generally effective for stabilizing the solution, they are beneficial in that they act as scavengers for the oxygen which is introduced into the solution during the preparation process.

The invention will now be further described with reference to the following illustrative but non-limiting examples.

### EXAMPLE

### BLEACH OIL

A bleach oil composition may be made with the allowing composition.

**Table**

| **Description** | **% By Weight** |
|---|---|
| Sodium Sulfite | 0.5 |
| Trisodium Phosphate | 1.5 |
| Maltose, Glucose or a combination | 5. |
| Isopropyl Alcohol | 9.6 |
| Ethanolamine | 5.3 |
| Oleic Acid | 7.1 |
| Octoxynol-9 | 8.3 |
| Lauramidopropyl Betaine | 9.3 |
| Cocamide DEA | 5.4 |
| Lauryl Alcohol | 11.4 |
| Propylene Glycol | 4.8 |
| Disodium EDTA | 0.25 |
| Sorbotol | 9.2 |
| Deionized Water | balance |

## Claims

1. A bleach oil composition for use in combination with an aqueous solution of sodium peroxide to form a hair bleaching product, the composition comprising:
A) from 0.1 to 1 wt.% sodium sulfite;
B) from 0.1 to 3 wt.% trisodium phosphate; and
C) from 0.2 to 12 wt.% of a reducing sugar.

2. The composition of claim 1 wherein said reducing sugar is selected from the group consisting of maltose, lactose, glucose, fructose or combinations thereof.

## Patentansprüche

1. Bleichölzusammensetzung zur Verwendung in Kombination mit einer wässrigen Lösung von Natriumperoxid, um ein Haarbleichprodukt zu bilden, wobei die Zusammensetzung umfasst:
A) 0,1-1 Gew.% Natriumsulfit,
B) 0,1-3 Gew.% Trinatriumphosphat und
C) 0,2-12 Gew.% reduzierenden Zucker.

2. Zusammensetzung nach Anspruch 1, wobei der reduzierende Zucker ausgewählt ist aus der Gruppe bestehend aus Maltose, Lactose, Glucose, Fructose oder Kombinationen davon.

## Revendications

1. Une composition d'huile de blanchiment pour une utilisation avec une solution aqueuse de peroxyde de sodium pour former un produit de blanchiment des cheveux, ladite composition comprenant:
A) de 0,1 à 1 % en poids de sulfite de sodium,
B) de 0,1 à 3 % en poids de phosphate trisodique
C) de 0,2 à 12 ù en poids de sucre réducteur.

2. Composition selon la revendication 1 **caractérisée en que** ledit sucre réducteur est choisi dans le groupe composé par le maltose, le lactose, le glucose, le fructose et leurs mélanges
